# DEMANDE DE BREVET EUROPEEN

(11) **EP 1 847 296 A2**
(43) Date de publication de la demande: **24.10.2007**
(21) Numéro de dépôt: 07105998.4
(22) Date de dépôt: 12.04.2007
(51) Int. Cl.: A61Q 5/06, A61K 8/40, A61K 8/69, A61K 8/70, A61K 8/31, A61K 8/897, A61K 8/86

(54) **Composition cosmétique comprenant au moins un monomère électrophile, au moins un composé fluoré et un solvant organique liquide.**

(30) Priorité: 13.04.2006 FR 0603281
(71) Demandeur: L'Oréal, 75008 Paris (FR)
(72) Inventeur: BRUN, Gaëlle, 75015, PARIS (FR); LIVOREIL, Aude, 75006, PARIS (FR)
(74) Mandataire: Dossmann, Gérard

(57) **Abrégé**

La présente invention concerne une composition cosmétique de traitement pour les fibres kératiniques, en particulier des fibres kératiniques humaines telles que les cheveux, comprenant au moins un solvant organique liquide, au moins un monomère électrophile, et au moins un composé fluoré organique contenant au moins 4 atomes de carbone, à l'exception du polytétrafluoroéthyléne et des pigments enrobés de composés fluorés.

## Description

La présente invention concerne une composition cosmétique de traitement des fibres kératiniques, en particulier des fibres kératiniques humaines telles que les cheveux, comprenant, au moins un monomère électrophile, au moins un composé fluoré organique particulier, et au moins un solvant organique liquide, une utilisation de cette composition pour le traitement des cheveux ainsi qu'un procédé de traitement la mettant en oeuvre.

Les cheveux sont généralement abîmés et fragilisés par l'action des agents atmosphériques extérieurs tels que la lumière et les intempéries, et par des traitements mécaniques ou chimiques tels que le brossage, le peignage, les décolorations, les permanentes et/ou les teintures. Il en résulte que les cheveux sont souvent difficiles à discipliner, en particulier ils sont difficiles à démêler ou à coiffer, et les chevelures, même abondantes, conservent difficilement une coiffure de bon aspect en raison du fait que les cheveux manquent de vigueur, de volume et de nervosité.

Ainsi, pour remédier à cela, il est usuel d'utiliser des produits de coiffage qui permettent de conditionner les cheveux en leur apportant notamment du corps, de la masse ou du volume.

Ces produits de coiffage sont généralement des compositions cosmétiques capillaires comprenant un ou plusieurs polymères qui présentent une forte affinité pour les cheveux et qui ont le plus souvent pour fonction de former un film à leur surface en vue de modifier leurs propriétés superficielles, notamment pour les conditionner.

Un inconvénient lié à l'utilisation de ces compositions capillaires réside dans le fait que les effets cosmétiques conférés par de telles compositions ont tendance à disparaître, notamment dès le premier shampooing.

Afin de remédier à cet inconvénient, il est envisageable d'accroître la rémanence du dépôt de polymères en effectuant directement une polymérisation radicalaire de certains monomères au niveau des cheveux. Toutefois, les traitements ainsi obtenus entraînent une dégradation de la fibre et les cheveux ainsi traités sont généralement difficilement démêlables.

Par ailleurs, il est connu du document FR 2 833 489 d'utiliser des monomères électrophiles polymérisant par voie anionique directement à la surface des cheveux en présence d'un agent nucléophile tel que des ions hydroxyde (OH⁻) contenus dans l'eau à pH neutre. Ainsi, une fois déposés sur la chevelure, ces monomères forment un polymère qui conduit à un gainage satisfaisant.

Cependant, le gainage obtenu à partir de ces compositions ne présente pas une résistance satisfaisante par rapport aux diverses agressions extérieures que peuvent subir les cheveux.

Il existe donc un réel besoin de trouver des compositions cosmétiques, notamment pour le conditionnement des cheveux, qui soient rémanentes aux shampooings et aux agressions extérieures tout en conservant de bonnes propriétés cosmétiques, c'est-à-dire d'apporter du corps, de la masse ou du volume aux cheveux et ceci de manière durable.

Ainsi la présente invention a pour objet une composition cosmétique pour le traitement des fibres kératiniques, en particulier des fibres kératiniques humaines telles que les cheveux, comprenant
- au moins un solvant organique liquide,
- au moins un monomère électrophile, et
- au moins un composé fluoré organique contenant au moins 4 atomes de carbone à l'exception du polytétrafluoroéthylène, et des pigments enrobés de composé fluorés.

Par l'application d'une telle composition, on obtient un gainage rémanent qui présente en particulier une bonne résistance aux shampooings et aux agressions extérieures telles que la transpiration, la lumière et les corps gras comme le sébum. Par ailleurs, la composition de l'invention permet d'obtenir une amélioration du corps, de la masse et du volume de la chevelure et ceci de manière durable.

Le gainage se présente aussi sous la forme d'un dépôt homogène, lisse et possédant une excellente adhésion sur le cheveu.

Dans le cadre de l'invention, les monomères électrophiles, le solvant organique et les composés fluorés sont des composés distincts.

L'invention a aussi pour objet un procédé de traitement cosmétique des fibres kératiniques mettant en oeuvre la composition cosmétique selon l'invention.

Un autre objet de la présente invention consiste en une utilisation de la composition cosmétique pour le traitement des fibres kératiniques.

L'invention a encore pour objet un dispositif à plusieurs compartiments ou kits comprenant d'une part une composition (C) comprenant au moins un monomère électrophile et d'autre part une composition (D) comprenant au moins un composé fluoré organique contenant au moins 4 atomes de carbone à l'exception du polytétrafluoroéthylène, et des pigments enrobés de composés fluorés l'une et/ou l'autre composition contenant au moins un solvant organique liquide.

D'autres objets, caractéristiques, aspects et avantages de l'invention apparaîtront encore plus clairement à la lecture de la description et des exemples qui suivent.

Le ou les monomères électrophiles présents dans la composition de l'invention peuvent être choisis parmi :
- les dérivés benzylidène malononitrile (A), le 2-(4-chloro-benzylidène)-malononitrile (A1), le 2-cyano-3-phényl acrylate d'éthyle (B), le 2-cyano-3-(4-chloro-phényl) acrylate d'éthyle (B1), décrits dans Sayyah, J. Polymer Research, 2000, p. 97 : 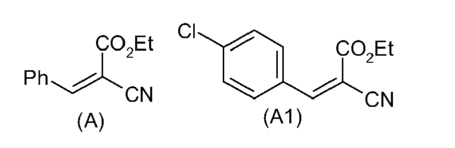 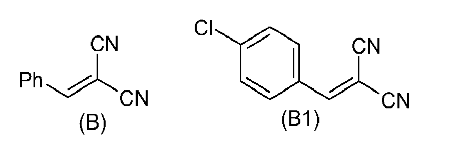
- les dérivés de méthylidènemalonates comme :
   ■ le 2-méthylène-malonate de diéthyle (C) décrit dans Hopff, Makromoleculare Chemie, 1961, p. 95, De Keyser, J. Pharm. Sci, 1991, p67 et Klemarczyk, Polymer, 1998, p. 173 : 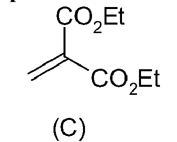
   ■ le 2-éthoxycarbonylméthylèneoxycarbonyl acrylate d'éthyle (D) décrit dans Breton, Biomaterials, 1998, p. 271 et Couvreur, Pharmaceutical Research, 1994, p. 1270 : 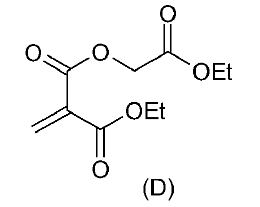
- les dérivés itaconate et itaconimide comme :
   ■ l'itaconate de diméthyle (E) décrit dans Bachrach, European Polymer Journal, 1976, p. 563 : 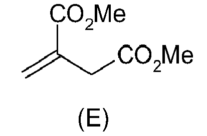
   ■ le N-butyl itaconimide (F), le N-(4-tolyl) itaconimide (G), le N-(2-éthylphényl) itaconimide (H), le N-(2,6-diéthylphényl) itaconimide (I), décrits par Wanatabe, J.Polymer Science : Part A : Polymer chemistry, 1994, p. 2073 : 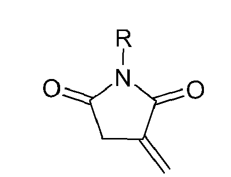
   R= Bu (F), 4-tolyl (G), 2-éthylphényl (H), 2,6-diéthyphényl (I)
- les dérivés α-(méthylsulfonyl)acrylates de méthyle (K), α-(méthylsulfonyl)acrylates d'éthyle (L), α-(tert-butylsulfonyl)acrylates de méthyle (M), α-(methylsulfonyl)acrylates de tert-butyle (N), α-( tert-butylsulfonyl)acrylates de tert-butyle (O), décrits dans Gipstein, J. Org. Chem, 1980, p. 1486 et
   les dérivés 1,1-bis-(méthylsulfonyl)éthylène (P), 1-acétyl-1-méthyl sulfonyl éthylène (Q), α-(méthylsulfonyl) vinyl sulfonate de méthyle (R), α-méthylsulfonylacrylonitrile (S), décrits dans le brevet US 2 748 050 : 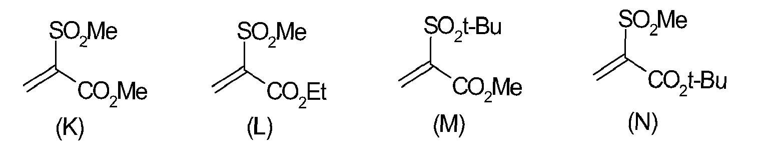 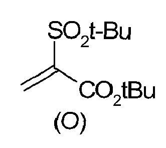 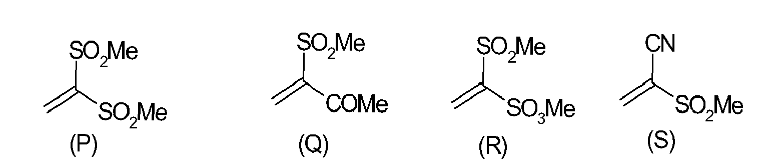
- les dérivés méthyl vinyl sulfone (T) et phényl vinyl sulfone (U) décrits dans Boor , J.Polymer Science, 1971, p. 249 : 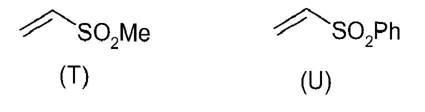
- le dérivé phényl vinyl sulfoxide (V) décrit dans Kanga, Polymer preprints (ACS, Divison of Polymer Chemistry), 1987, p. 322 : 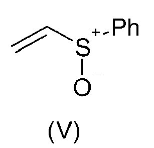
- le dérivé 3-méthyl-N-(phénylsulfonyl)-1-aza-1,3-butadiène (W) décrit dans Bonner, Polymer Bulletin, 1992, p. 517 : 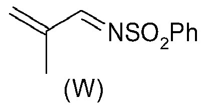
- les dérivés acrylates et acrylamides comme :
   ■ le N-propyl-N-(3-triisopropoxysilylpropyl)acrylamide (X) et le N-propyl-N-(3-triethoxysilylpropyl)acrylamide (Y) décrits dans Kobayashi, Journal of Polymer Science, Part A : Polymer Chemistry, 2005, p. 2754 : 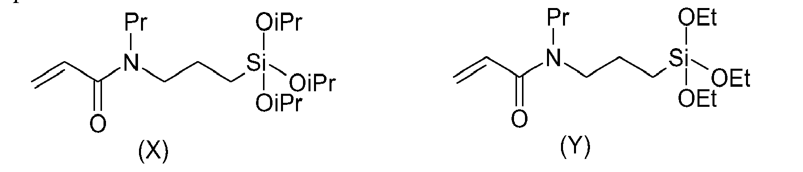
   ■ le 2-hydroxyéthyl acrylate (Z) et le 2-hydroxyéthyl méthacrylate (AA) décrits dans Rozenberg, International Journal of Plastics Technology, 2003, p. 17 : 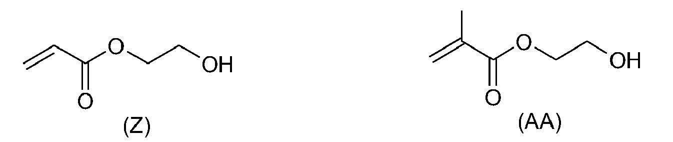
   ■ le N-butyl acrylate (AB) décrit dans Schmitt, Macromolecules, 2001, p. 2115 et
      le tert-butyl acrylate (AC) décrit dans Ishizone, Macromolecules, 1999, p. 955 :

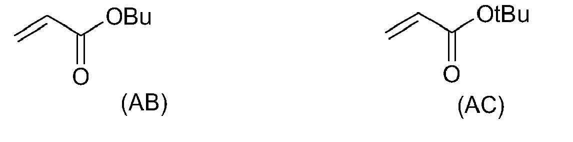

Le monomère électro-attracteur utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier, ces monomères présentent au moins deux groupes électro-attracteurs.

A titre d'exemple de monomères présentant au moins deux groupes électro-attracteurs, on peut citer les monomères de formule (I) : 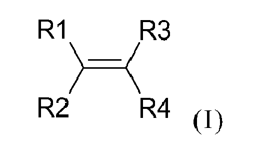 dans laquelle :
R1 et R2 désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, - SR, -OH, et les atomes d'halogène,
   - un résidu polyorganosiloxane modifié ou non,
   - un groupement polyoxyalkylène,
R3 et R4 désignent chacun, indépendamment l'un de l'autre, un groupe électro-attracteur (ou inductif-attacteur) choisi de préférence parmi les groupements -N(R)₃⁺, -S(R)₂⁺, -SH₂⁺, -NH₃⁺, -NO₂, -SO₂R, -C≡N, -COOH, -COOR, -COSR, -CONH₂, CONHR, -F, -Cl, -Br, -I, - OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle tels que phényle, ou les groupements aryloxy tels que phénoxyloxy,
R désigne un groupe hydrocarboné saturé ou non, linéaire, ramifié ou cyclique, comportant de préférence de 1 à 20 atomes de carbones, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C₁-C₁₀.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et / ou siloxy et/ou silanol et / ou amine et / ou imine et / ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)ₙ-(CF₂)ₘ-CF₃ ou - (CH₂)ₙ-(CF₂)ₘ-CHF₂ avec n = 1 à 20 et m = 1 à 20.

Les substituants R1 à R4 peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthane.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et de type esters gras.

Parmi les monomères précédemment cités, sont préférés les monomères de la famille des cyanoacrylates et leurs dérivés de formule (II) : 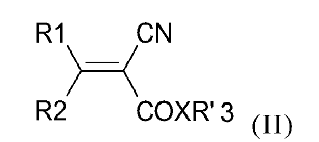 dans laquelle :
X désignant NH, S ou O,
R1 et R2 ayant les mêmes significations que précédemment, de préférence R1 et R2 représentant un atome d'hydrogène,
R'3 représentant un atome d'hydrogène ou R tel que défini à la formule (I).

De préférence, X désigne O.

A titre de composés de formule (II), on peut citer les monomères :
a) appartenant à la famille des 2-cyanoacrylates de polyfluoroalkyle tels que l'ester 2,2,3,3-tétrafluoropropylique de l'acide 2-cyano-2-propénoïque de formule (III) : 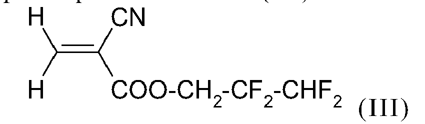 ou encore l'ester 2,2,2-trifluoroéthylique de l'acide 2-cyano-2-propénoïque de formule (IV) : 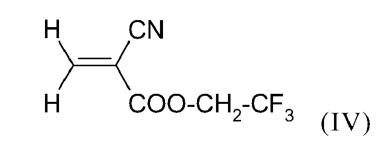
b) les 2-cyanoacrylates d'alkyle ou d'alcoxyalkyle de formule (V) : 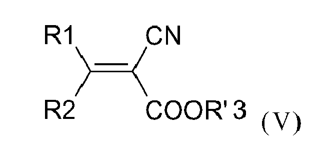 dans laquelle :
   R1 et R2 sont tels que définis précédemment,
   R'3 représente un radical alkyle en C₁-C₁₀, alcoxy(C₁-C₄) alkyle(C₁-C₁₀) ou alcényle en C₂-C₁₀,

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle, le 2-cyanoacrylate d'allyle, le 2-cyanoacrylate de méthoxypropyle et le cyanoacrylate d'iso-amyle.

Dans le cadre de l'invention, on préfère utiliser les monomères définis en b). Selon un mode de réalisation préféré, le ou les monomères cyanoacrylates sont choisis parmi les cyanoacrylates d'alkyle en C₆-C₁₀.

Les monomères particulièrement préférés sont les cyanoacrylates d'octyle de formule (VI) et leurs mélanges : 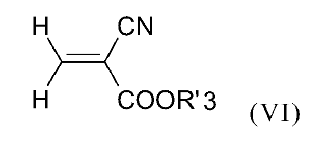 dans laquelle R'3 est choisi parmi les radicaux suivants :
-(CH₂)₇-CH₃ ;
-CH(CH₃)-(CH₂)₅-CH₃ ;
-CH₂-CH(C₂H₅)-(CH₂)₃-CH₃ ;
-(CH₂)₅-CH(CH₃)-CH₃ ;
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

De préférence, les monomères électrophiles sont présents dans une quantité allant de 0,2 à 50 % en poids, et encore plus préférentiellement dans une quantité allant de 0,5 à 30 % en poids, par rapport au poids total de la composition cosmétique.

Dans le cadre de la présente invention, les monomères électrophiles de formule (I) sont des monomères capables de polymériser par voie anionique en présence d'un agent nucléophile.

Par polymérisation anionique, on entend le mécanisme défini dans l'ouvrage "Advanced Organic Chemistry", Third Edition de Jerry March, pages 151 à 161.

Les agents nucléophiles susceptibles d'initier la polymérisation anionique sont des systèmes connus en eux-mêmes, capables de générer un carbanion au contact d'un agent nucléophile, tels que les ions hydroxydes contenus dans l'eau à pH neutre. On entend par " carbanion ", les espèces chimiques définies dans " Advanced Organic Chemistry, Third Edition ", de Jerry March, page 141.

Les agents nucléophiles peuvent être appliqués indépendamment de la composition de l'invention. Ils peuvent aussi être ajoutés à la composition de l'invention au moment de l'emploi.

L'agent nucléophile est un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PHNH⁻, pyridine, ArS⁻, R-C^{≡}C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻, H₂O, Ph représentant un groupe phényle ; Ar représentant un groupe aryle et R représentant un groupe alkyle en C₁-C₁₀.

Les monomères électrophiles de formule (I) selon la présente invention peuvent être synthétisés selon les méthodes connues décrites dans la technique. En particulier, les monomères cyanoacrylates peuvent être synthétisés selon l'enseignement du US 3 527 224, US 3 591 767, US 3 667 472, US 3 995 641, US 4 035 334 et US 4 650 826.

Au sens de la présente invention, on entend par composé fluoré tout composé organique comprenant au moins un atome de fluor, et contenant au moins 4 atomes de carbone. De manière générale, on préférera les composés perfluorés c'est-à-dire les composés pour lesquels les atomes d'hydrogène associés à au moins un atome de carbone sont remplacés totalement par des atomes de fluor.

Ce composé fluoré peut être sous la forme d'un composé défini ou d'un polymère, d'une silicone, d'un copolymère.

De telles composés sont notamment décrits dans l'encyclopédie de chimie industrielle Ullmann dans les chapitres fluoropolymères (fluoroplastics, fluoroelastomers) et fluorine compounds, organic.

De préférence le composé fluoré est différent d'un polymère silicone greffé à base d'acrylate.

Selon un mode de réalisation particulier, lorsque le composé fluoré est liquide à 25°C et pression atmosphérique, alors le solvant organique liquide n'est pas fluoré.

Parmi les composés fluorés répondant à la définition, on peut citer :
i) les composés fluorosiliconés de formule (VII) : 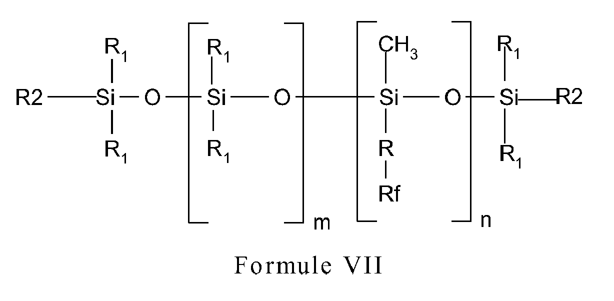 dans laquelle :
   - R représente un groupement divalent alkyle linéaire ou ramifié, ayant 1 à 6 atomes de carbone, de préférence un groupement divalent méthyle, éthyle, propyle ou butyle,
   - Rf représente un radical fluoroalkyle, notamment un radical perfluoroalkyle, ayant 1 à 12 atomes de carbone, de préférence 1 à 9 atomes de carbone,
   - R₁ représente, indépendamment l'un de l'autre, un radical alkyle en C₁-C₂₀, un radical hydroxyle, un radical phényle,
   - R₂ représente R₁ ou R_{f}
   - m est choisi de 0 à 500, de préférence de 0 à 200, et
   - n est choisi de 1 à 1000, de préférence de 1 à 500.
   De préférence, les groupements R₁ sont identiques et représentent un radical méthyle.
   De tels composés sont notamment ceux commercialisés par la société Shin Etsu sous les dénominations 'FL-5', 'FL-10', 'X22-821' et 'X22-822' ou encore 'FL-100', par la société Dow Corning sous le nom FS-1265 Fluid, par la société Phoenix Chemical sous la gamme Pecosil FS sous les dénominations Pecosil FSL-150, Pecosil FSL-300, Pecosil FSH-150, Pecosil FSH-300, Pecosil FSU-150, Pecosil FSU-300.
ii) les composés perfluorocycloalkyles.
   On peut notamment citer les perfluorocycloalkyles répondants à la formule (VIII) suivante : 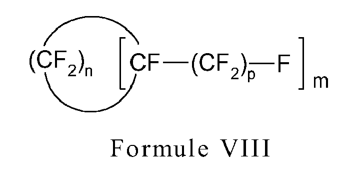 dans laquelle n est égal à 3, 4 ou 5, m est égal à 1 ou 2, et p est égal à 0, 1, 2 ou 3 ; sous réserve que lorsque m=2, les groupements ne sont pas nécessairement en alpha l'un par rapport à l'autre.
   Parmi les composés de formule (VIII), on peut notamment citer le perfluorométhylcyclopentane, et le 1,3-perfluorodiméthylcyclohexane, vendus respectivement sous les dénominations de "FLUTEC PC1^{®}" et "FLUTEC PC3^{®}" par la Société F2 CHEMICALS, ainsi que le perfluorodiméthylcyclobutane.
   Parmi les composés perfluorocycloalkyles, on peut aussi citer les composés polycycliques. On peut notamment citer le perfluorodecaline, et le perfluoroperhydrophenanthrene, vendus respectivement sous les dénominations de "FLUTEC PC6^{®}" et "FLUTEC PC11^{®}" par la Société F2 CHEMICALS.
iii) les composés fluoroalkyles ou hétérofluoroalkyles répondant à la formule (IX) suivante :

   CH₃-(CH₂)ₙ-[Z]ₜ-X-CF₃ (IX) Formule IX

   dans laquelle t est 0 ou 1 ; n est 0, 1, 2 ou 3 ; X est un radical perfluoroalkyle divalent, linéaire ou ramifié, ayant de 2 à 5 atomes de carbone, et Z représente O, S, ou NR, R étant hydrogène, un radical - (CH₂)ₙ-CH₃ ou -(CF₂)ₘ-CF₃, m étant 2, 3, 4 ou 5.
   Parmi les composés fluoroalkyles ou hétérofluoroalkyles de formule (IX) on peut notamment citer le méthoxynonafluorobutane vendu sous la dénomination de "MSX 4518^{®}", "HFE-7100^{®}" par la Société 3M et l'éthoxynonafluorobutane vendu sous la dénomination de "HFE-7200^{®}" par la Société 3M.
iv) les composés perfluoroalcanes répondant à la formule (X) suivante :

   CF₃-(CF₂)ₙ-CF₃ Formule (X)

   dans laquelle n vaut de 2 à 16, de préférence de 2 à 6.
   Parmi les composés perfluoroalcanes de formule (X) on peut notamment citer le perfluorohexane, le dodécafluoropentane, le tétradécafluorohexane.
v) les perfluoropolyéthers répondant aux formules (XI) et (XII) suivantes : 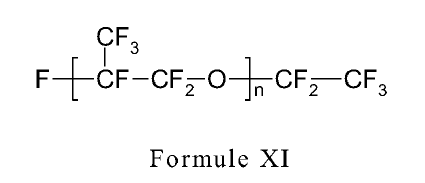 dans laquelle n vaut de 7 à 30 ; et 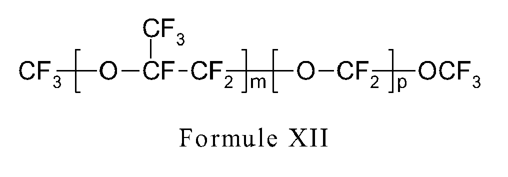 le rapport m/p étant de 20 à 40, et le poids moléculaire allant de 500 à 20000.
   Parmi ces perfluoropolyéthers de formules (XI) et (XII), on peut respectivement citer celui vendu sous la dénomination de "FLUORTRESS LM36^{®}" par la Société DUPONT, et ceux vendus sous la dénomination générale de "FOMBLIN" par la Société MONTEFLUOS par exemple FOMBLIN HC R^{®}.
   On peut également utiliser les perfluoropolyéthers cités dans la demande EP-A-641194 dont le contenu est incorporé, à titre de référence, dans la présente demande.
vi) les dérivés de perfluomorpholine répondant à la formule (XIII) suivante : 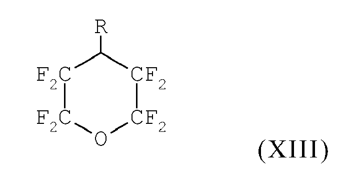 dans laquelle R représente un radical perfluoroalkyle en C₁-C₄.
   Parmi les dérivés de perfluoromorpholine de formule (XIII), on peut notamment citer la 4-trifluorométhyl perfluoromorpholine et la 4-pentafluoroéthyl perfluoromorpholine.
vii) Les esters fluorés répondant à la formule (XIV) suivante : 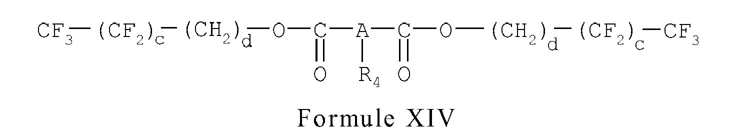 dans laquelle :
   R₄ représente un atome d'hydrogène ou le radical 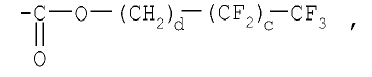
   A représente une chaîne alkylène ou alcénylène, linéaire ou ramifiée, éventuellement hydroxylée, en C₁-C₁₈,
   c est 1 à 17, et
   d est 1 à 18.
   Parmi les composés de formule (XIV), on peut notamment citer le dodécane 1,12,dioate de 2F-octyl-éthyle de formule suivante : 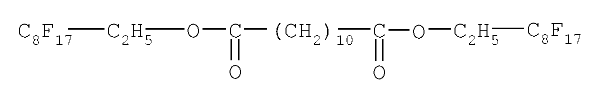 et les tri-citrates de perfluoroalkyle en C₁-C₆, plus particulièrement le tri-citrate de perfluorobutyle vendu sous la dénomination de "Zonyl TBC®" par la Société Dupont.
viii) les copolymères à base de perfluoroalkylpolyacrylate.
   De façon avantageuse, la partie du copolymère contenant le perfluoroalkylpolyacrylate est formé par la polymérisation d'un monomère de type acrylique ou de type acrylamide présentant en particulier la formule (A') suivante : 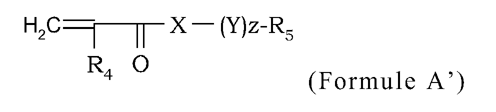 dans laquelle :
   - R₄ représente un atome d'hydrogène ou un radical alkyle en C₁-C₄,
   - X représente O ou -N-R₂, pour lequel R₂ représente un atome d'hydrogène ou un radical alkyle ou hydroxyalkyle en C₁-C₁₀.
   - Y représente un radical alkylène en C₁-C₆
   - z est égal à 0 ou 1,
   - R₅ représente un radical alkyle linéaire ou ramifiée en C₁-C₂₀ dont tout ou partie des atomes d'hydrogène est remplacé par des atomes de fluor ou un radical contenant des groupements perfluoropolyéthers.

A titre d'exemple, le monomère fluoré peut être de formule : 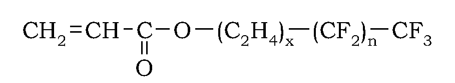 avec n allant de 4 à 16, de préférence égal à 5, 7, 9 ou 11; et x égal à 1 ou 2.

Le monomère fluoré peut aussi être de formule : 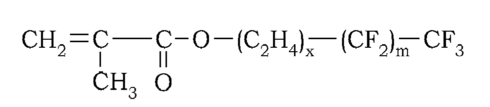 avec m allant de 4 à 16, de préférence égal à 5, 7, 9 ou 11; et x égal à 1 ou 2.

La partie non fluorée du copolymère peut être notamment issue des monomères de type B' ou C'utilisés seuls ou en association :

### Monomère B' :

Monomère à base d'alkyl(méth)acrylate répondant à la formule (B') : 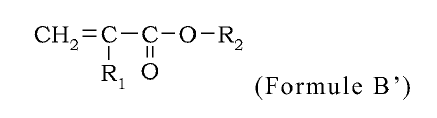 dans laquelle :
- R₁ représente un atome d'hydrogène ou un radical alkyle en C₁-C₄ et
- R₂ représente un radical alkyle en C₁-C₂₀, de préférence C₂-C₈, un radical hydroxycarboné en C₂-C₆, de préférence C₂-C₄, ou un radical -(CH₂)ₙ-NH-R₃ avec R₃ représentant un alkyle en C₁-C₆ ou un cycloalkyle et n étant un entier allant de 1 à 4.

De préférence, on choisit le radical R₁ du monomère de formule B' parmi l'hydrogène ou un radical méthyle, éthyle, n-butyle ou isopropyle.

De préférence, on choisit les monomères de formule B' parmi l'acide acrylique, l'acide méthacrylique, le (méth)acrylate de méthyle, le (méth)acrylate d'éthyle, le (méth)acrylate de butyle, le (méth)acrylate d'isobutyle, le (méth)acrylate d'éthyl-2 hexyle, le (méth)acrylate de lauryle, le (méth)acrylate d'hydroxyéthyle, le (méth)acrylate de 2-hydroxypropyle, leurs mélanges.

En particulier, les copolymères fluorés de l'invention peuvent être fabriqués comme décrit dans les documents FR-A-2175332, FR-A-2540131 et EP-A-206671.

Ces copolymères sont par exemple ceux vendus par Cognis sous la marque Repellan, ceux vendus par Mitsubishi Chemicals sous la marque Repearl, ou ceux vendus par Clariant sous le nom Cartafluor UH Liquide.

### Monomère C' :

Monomère comprenant un motif alkyl(méth)acrylamide répondant à la formule C' : 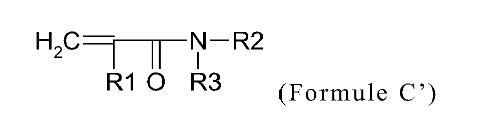 dans laquelle R1 représente H ou CH₃ ;
R2 et R3, identiques ou différents, représentent H, un radical hydrocarboné linéaire ou ramifié, saturé ou insaturé, en C₁-C₄₀, éventuellement substitué par un ou plusieurs groupes hydroxyle, acide carboxylique, acide sulfonique ou acide phosphonique, estérifié ou non, un groupe cycloalkyle en C₅-C₁₀ ou aryle en C₅-C₁₀, éventuellement substitué par un ou plusieurs groupes alkyle, linéaires ou ramifiés, en C₁-C₄, hydroxyle, acide carboxylique ou acide sulfonique ou pouvant comporter, à ses extrémités ou à l'intérieur, un ou plusieurs hétéroatomes choisis parmi O, S, N, Si et P ou un groupe carbonyle, ou pouvant comporter un ou plusieurs hétéroatomes choisis parmi O, S et N ;
Parmi les motifs (méth)acrylamide préférés, on peut citer les motifs N-tertiobutylacrylamide, N-tertiohexylacrylamide, N-tertiooctylacrylamide, N-octylacrylamide, N-décylacrylamide, N-dodécylarylamide et N-méthlyundécylacrylamide.
ix) Composé fluoré contenant la sous-unité suivante : 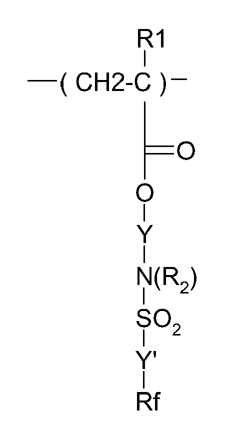 dans laquelle :
   - R₁ représente un atome d'hydrogène ou un radical alkyle en C₁-C₄,
   - R₂ représente un atome d'hydrogène ou un radical alkyle ou hydroxyalkyle en C₁-C₁₀.
   - Y et Y' représentent des radicaux alkylène en C₁-C₆
   - Rf représente un radical alkyle linéaire ou ramifiée en C₁-C₂₀ dont tout ou partie des atomes d'hydrogène est remplacé par des atomes de fluor ou un radical contenant des groupements perfluoropolyéthers.
   En particulier, les copolymères fluorés de l'invention peuvent être synthétisés comme décrit dans les documents FR-A-2175332, FR-A-2540131.
   Des exemples de ces copolymères peuvent être trouvés dans la gamme Scotchgard commercialisée par 3M, dont la formule est la suivante : 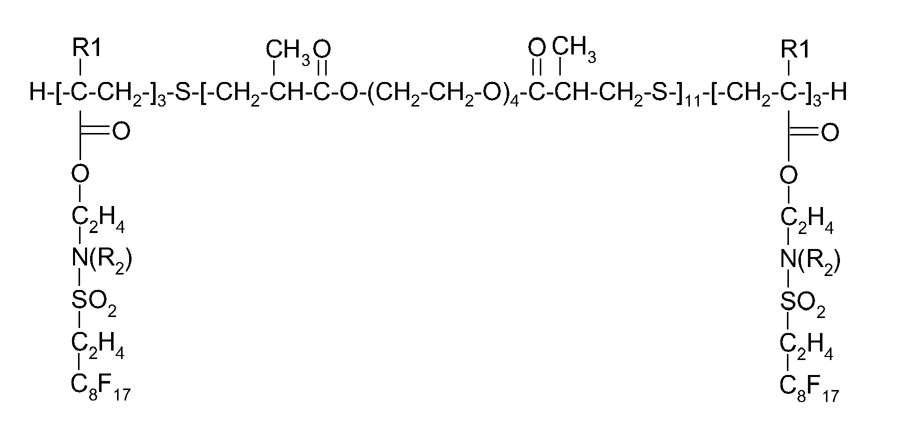 Ou dans la gamme Foraperle commercialisée par la société Atochem dont la structure est la suivante : 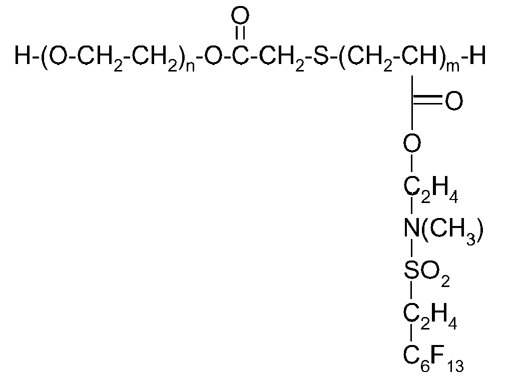
x) Le composé fluoré peut aussi être un copolymère à base de Fluoroethylène - alkylvinyl ether. Comme composé appartenant à cette famille, on peut notamment citer la gamme Lumiflon commercialisée par Asahi Glass.
xi) On peut également utiliser comme huiles fluorées les fluorohydrocarbures cités dans la demande EP-A-609132 dont le contenu est incorporé à titre de référence dans la présente demande. Comme composé appartenant à cette famille, on peut notamment citer le 1-(2'-fluorohexylethylthio)-3-(2"-ethylhexyloxy)-2-propanol.

Les composés fluorés sont choisis de préférence parmi les composés fluorosiliconés, les perfluorocycloalkyles, les perfluoropolyéthers, les copolymères à base de perfluoroalkylpolyacrylate.

Ces composés fluorés peuvent être solubles ou en dispersion dans le milieu de la composition. Ils peuvent aussi se présenter sous forme de particules en dispersion, la dispersion contenant éventuellement un alcool et/ou une cétone en faible quantité, dispersion que l'on peut diluer en toute proportion dans l'eau.

Les composés fluorés sont présents dans la composition cosmétique selon l'invention dans une quantité allant généralement de 0,1 à 20 % en poids, de préférence dans une quantité allant de 0,2 % à 15 % en poids, et plus préférentiellement encore dans une quantité allant de 0,5 à 10 % en poids, par rapport au poids total de ladite composition cosmétique.

Par solvant organique, on entend une substance organique capable de dissoudre une autre substance sans la modifier chimiquement.

Les solvants organiques liquides utiles dans la composition cosmétique selon l'invention sont choisis parmi les composés liquides à la température de 25°C et sous 105 Pa (760mm de Hg).

Le solvant organique est par exemple choisi parmi :
les alcools aromatiques tels que l'alcool benzylique ; les alcools gras liquides, notamment en C₁₀-C₃₀; les polyols modifiés ou non tels que le glycérol, le glycol, le propylène glycol, le dipropylène glycol, le butylène glycol, le butyle diglycol ; les silicones volatiles telles que la cyclopentasiloxane, la cyclohexasiloxane, les polydiméthylsiloxanes modifiées ou non par des fonctions alkyle et/ou amine et/ou imine et/ou fluoroalkyl et/ou carboxylique et/ou betaïne et/ou ammonium quaternaire, les polydiméthylsiloxanes modifiées liquides, les huiles minérales, organiques ou végétales, les alcanes et plus particulièrement les alcanes de C₅ à C_{10 ;} les acides gras liquides, les esters gras liquides et plus particulièrement les benzoates ou les salicylates d'alcool gras liquides.

Le solvant organique est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ou encore des composés organiques tels que des alcanes en C₅-C₁₀, l'acétone, la méthyléthylcétone, les esters d'acides en C₁-C₂₀ liquides et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras liquides en C₁₀-C₃₀ tels que l'alcool oléique, les esters d'alcools gras en C₁₀-C₃₀ liquides tels que les benzoates d'alcool gras en C₁₀-C₃₀ et leurs mélanges ; l'huile de polybutène, l'isononanoate d'isononyle, le malate d'isostéaryle, le tétra-isostéarate de pentaérythrityle, le trimélate de tridécyle, le mélange cyclopentasiloxane (14,7% en poids)/polydiméthylsiloxane dihydroxylé en positions alpha et oméga (85,3% en poids), ou leurs mélanges.

Selon un mode de réalisation particulier, le solvant organique est non fluoré.

Selon un mode de réalisation préféré, le solvant organique liquide est constitué par une silicone ou un mélange de silicones tels que les polydiméthylsiloxanes liquides et les polydiméthylsiloxanes modifiées liquides, leur viscosité à 25°C est comprise entre 0,1cst et 1 000 000cst et plus préférentiellement entre 1 cst et 30 000cst.

On citera de préférence les huiles suivantes :
- le mélange de polydiméthylsiloxane alpha-omega-dihydroxylé/cyclopentadiméthylsiloxane (14,7/85,3) commercialisé par Dow Corning sous le nom de DC 1501 Fluid
- le mélange de polydiméthylsiloxane alpha-omega-dihydroxylé/ polydiméthylsiloxane commercialisé par Dow Corning sous le nom de DC 1503 Fluid
- le mélange de diméthicone /cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC245 Fluid ;
et les mélanges respectifs de ces huiles.

La composition de l'invention peut contenir de l'eau. Cependant, cette composition est de préférence anhydre c'est-à-dire contenant moins de 1% en poids d'eau par rapport au poids total de la composition.

Le ou les solvants organiques liquides de la composition représentent généralement de 0,01 à 99 %, de préférence de 50 à 99 % en poids par rapport au poids total de la composition.

La composition cosmétique selon l'invention peut comprendre en outre au moins un pigment.

L'utilisation d'un pigment dans la composition cosmétique conforme à l'invention permet d'obtenir des colorations visibles même sur des cheveux foncés sans qu'il soit nécessaire de décolorer les fibres kératiniques puisque le pigment en surface masque la couleur naturelle de la fibre.

La composition conforme à l'invention présente ainsi l'avantage de conduire à des colorations qui présentent une bonne résistance aux diverses agressions que peuvent subir les cheveux, telles que les corps gras ou les shampooings successifs.

Ainsi, il est possible d'obtenir des colorations visibles et très chromatiques sur une fibre kératinique foncée sans qu'il soit nécessaire d'éclaircir ou de décolorer les fibres kératiniques et, par conséquent, sans dégradation physique des fibres kératiniques.

Au sens de la présente invention, on entend par pigment toute entité organique et / ou minérale dont la solubilité dans l'eau est inférieure à 0,01 % à 20 °C, de préférence inférieure à 0,0001 %, et présentant une absorption entre 350 et 700 nm, de préférence une absorption avec un maximum.

Les pigments qui peuvent être utilisés sont notamment choisis parmi les pigments organiques et/ ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments conformes à l'invention peuvent par exemple être choisis parmi les pigments blancs ou colorés, les laques, les pigments à effets spéciaux tels que les nacres ou les paillettes, et leurs mélanges.

A titre d'exemples de pigments minéraux blancs ou colorés, on peut citer le dioxyde de titane, traité ou non traité en surface, les oxydes de zirconium ou de cérium, les oxydes de fer ou de chrome, le violet de manganèse, le bleu outremer, l'hydrate de chrome et le bleu ferrique. Par exemple, les pigments minéraux suivants peuvent être utilisés : Ta₂O₅, Ti₃O₅, Ti₂O₃, TiO, ZrO₂ en mélange avec TiO₂, ZrO₂, Nb₂O₅, CeO₂, ZnS

A titres d'exemples de pigments organiques blancs ou colorés, on peut citer les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références Cl 42090, 69800, 69825, 73000, 74100, 74160, les pigments jaunes codifiés dans le Color Index sous les références Cl 11680, 11710, 15985, 19140, 20040, 21100, 21108, 47000, 47005, les pigments verts codifiés dans le Color Index sous les références C1 61565, 61570, 74260, les pigments oranges codifiés dans le Color Index sous les références CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

On peut utiliser des pâtes pigmentaires de pigment organique telles que les produits vendus par la société HOECHST sous le nom :
- JAUNE COSMENYL IOG : Pigment YELLOW 3 (C1 11710) ;
- JAUNE COSMENYL G : Pigment YELLOW 1 (C1 11680) ;
- ORANGE COSMENYL GR : Pigment ORANGE 43 (C1 71105) ;
- ROUGE COSMENYL R : Pigment RED 4 (C1 12085) ;
- CARMIN COSMENYL FB : Pigment RED 5 (C1 12490) ;
- VIOLET COSMENYL RL : Pigment VIOLET 23 (C1 51319) ;
- BLEU COSMENYL A2R : Pigment BLUE 15.1 (C1 74160) ;
- VERT COSMENYL GG : Pigment GREEN 7 (C1 74260) ;
- NOIR COSMENYL R : Pigment BLACK 7 (C1 77266).

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Par laque, on entend les colorants adsorbés sur des particules insolubles, l'ensemble ainsi obtenu restant insoluble lors de l'utilisation. Les substrats inorganiques sur lesquels sont adsorbés les colorants sont par exemple l'alumine, la silice, le borosilicate de calcium et de sodium ou le borosilicate de calcium et d'aluminium, et l'aluminium. Parmi les colorants organiques, on peut citer le carmin de cochenille.

A titre d'exemples de laques, on peut citer les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 7 (CI 15 850:1), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments blancs ou colorés qui procurent une teinte uniforme opaque, semi-transparente ou transparente classique.

A titre d'exemples de pigments à effets spéciaux, on peut citer les pigments nacrés blancs tels que le mica recouvert de titane, ou d'oxychlorure de bismuth, les pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre de pigments nacrés, on peut citer les nacres Cellini commercialisée par Engelhard (Mica-TiO2-laque), Prestige commercialisée par Eckart (Mica-TiO2), Prestige Bronze commercialisée par Eckart (Mica-Fe2O3),Colorona commercialisée par Merck (Mica-TiO2-Fe2O3).

On peut également citer les pigments à effet interférentiel non fixés sur un substrat comme les cristaux liquides (Helicones HC de Wacker), les paillettes holographiques interférentielles (Géométrie Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être synthétisées selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size séries of highly luminescent nanocristallites" Journal of phisical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

La variété des pigments qui peuvent être utilisés dans la présente invention permet d'obtenir une riche palette de couleurs, ainsi que des effets optiques particuliers tels que des effets métalliques, interférentiels.

Selon un mode de réalisation particulier, les pigments sont des pigments colorés. On entend par pigment coloré des pigments autres que les pigments blancs.

La taille du pigment utilisé dans la composition cosmétique selon la présente invention est généralement comprise entre 10 nm et 200 µm, de préférence entre 20 nm et 80 µm, et plus préférentiellement entre 30 nm et 50 µm.

Les pigments utilisés dans la composition cosmétique selon l'invention peuvent être traités en surface par un agent organique.

Ainsi les pigments préalablement traités en surface utiles dans le cadre de l'invention sont des pigments qui ont subi totalement ou partiellement un traitement de surface de nature chimique, électronique, électro-chimique, mécano-chimique ou mécanique, avec un agent organique tel que ceux qui sont décrits notamment dans Cosmetics and Toiletries, Février 1990, Vol. 105, p. 53-64 avant d'être dispersés dans la composition conforme à l'invention. Ces agents organiques peuvent être par exemple choisis parmi les acides aminés ; les cires, par exemple la cire de carnauba et la cire d'abeille ; les acides gras, les alcools gras et leurs dérivés, tels que l'acide stéarique, l'acide hydroxystéarique, l'alcool stéarylique, l'alcool hydroxystéarylique, l'acide laurique et leurs dérivés ; les tensio-actifs anioniques ; les lécithines ; les sels de sodium, potassium, magnésium, fer, titane, zinc ou aluminium d'acides gras, par exemple le stéarate ou laurate d'aluminium ; les alcoxydes métalliques ; les polysaccharides, par exemple le chitosane, la cellulose et ses dérivés ; le polyéthylène ; les polymères (méth)acryliques, par exemple les polyméthylmethacrylates ; les polymères et copolymères contenant des motifs acrylates ; les protéines ; les alcanoamines ; les composés siliconés, par exemple les silicones, les polydiméthylsiloxanes, les alcoxysilanes, les alkylsilanes, les siloxy-silicates ; les composés organiques fluorés, par exemple les perfluoroalkyle éthers ; les composés fluoro-siliconés.

Les pigments traités en surface utiles dans la composition cosmétique selon l'invention peuvent aussi avoir été traités par un mélange de ces composés et / ou avoir subi plusieurs traitements de surface.

Les pigments traités en surface utiles dans le cadre de la présente invention peuvent être préparés selon des techniques de traitement de surface bien connues de l'homme de l'art ou trouvés tels quels dans le commerce.

De préférence, les pigments traités en surface sont recouverts par une couche organique.

L'agent organique avec lequel sont traités les pigments peut être déposé sur les pigments par évaporation de solvant, réaction chimique entre les molécules de l'agent de surface ou création d'une liaison covalente entre l'agent de surface et les pigments.

Le traitement en surface peut ainsi être réalisé par exemple par réaction chimique d'un agent de surface avec la surface des pigments et création d'une liaison covalente entre l'agent de surface et les pigments ou les charges. Cette méthode est notamment décrite dans le brevet US 4 578 266.

De préférence, on utilisera un agent organique lié aux pigments de manière covalente.

L'agent pour le traitement de surface peut représenter de 0,1 à 50 % en poids du poids total des pigments traités en surface, de préférence de 0,5 à 30 % en poids, et encore plus préférentiellement de 1 à 10 % en poids.

De préférence, les traitements en surface des pigments sont choisis parmi les traitements suivants :
- un traitement PEG-Silicone comme le traitement de surface AQ commercialisé par LCW ;
- un traitement Chitosane comme le traitement de surface CTS commercialisé par LCW ;
- un traitement Triéthoxycaprylylsilane comme le traitement de surface AS commercialisé par LCW ;
- un traitement Méthicone comme le traitement de surface SI commercialisé par LCW ;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Lauroyl Lysine comme le traitement de surface LL commercialisé par LCW ;
- un traitement Lauroyl Lysine Diméthicone comme le traitement de surface LL / SI commercialisé par LCW ;
- un traitement Myristate de Magnésium comme le traitement de surface MM commercialisé par LCW ;
- un traitement Dimyristate d'Aluminium comme le traitement de surface MI commercialisé par Miyoshi ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Diméthicone / Disodium Stéaroyl Glutamate comme le traitement de surface SA / NAI commercialisé par Miyoshi ;
- un traitement Copolymère acrylate / Diméthicone et Phosphate de Perfluoalkyle comme le traitement de surface FSA commercialisé par Daito ;
- un traitement Lauryl Lysine / Aluminium Tristéarate comme le traitement de surface LL-StAI commercialisé par Daito ;
- un traitement Octyltriéthylsilane comme le traitement de surface OTS commercialisé par Daito ;
- un traitement Octyltriéthylsilane / Phosphate de Perfluoroalkyle comme le traitement de surface FOTS commercialisé par Daito ;
- un traitement Copolymère Acrylate / Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement Cellulose comme le traitement de surface C2 commercialisé par Daito ;
- un traitement copolymère Acrylate comme le traitement de surface APD commercialisé par Daito ;

Le ou les pigments sont généralement présents dans la composition dans des quantités totales généralement comprises entre 0,05 et 50 % en poids du poids total de la composition, de préférence entre 0,1 et 35 % en poids, encore plus préférentiellement entre 0,5 et 20 % en poids.

La composition conforme à la présente invention peut de plus comprendre un ou plusieurs pigments non traités en surface.

Le milieu de la composition de l'invention peut aussi se présenter sous forme d'une émulsion et / ou être encapsulé, les monomères électrophiles étant maintenus dans un milieu anhydre jusqu'au moment de l'utilisation. Lorsque le milieu est une émulsion, cette émulsion est par exemple constituée par une phase dispersée ou continue qui peut être constituée par de l'eau, des alcools aliphatiques en C1-C4 ou leurs mélanges et une phase organique anhydre comprenant le monomère. Dans le cas des capsules ou microcapsules, la capsule peut contenir le monomère dans un milieu anhydre et être dispersées dans un milieu anhydre tel que défini précédemment, de l'eau, des alcools aliphatiques en C1-C4 ou leurs mélanges.

On peut introduire dans les compositions des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, le trifluorure de bore, l'hydroquinone et ses dérivés tels que l'hydroquinone monéthyléther, la TBHQ, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, le 3-sulfonène et leurs mélanges. Les groupements alkyle désignent de préférence des groupement ayant 1 à 6 atomes de carbone.

On peut aussi utiliser à titre d'inhibiteur les acides minéraux ou organiques.

Ainsi la composition cosmétique selon l'invention peut également comprendre au moins un acide minéral ou organique, ce dernier ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique, l'acide octanoïque, l'acide heptanoïque et l'acide hexanoïque.

De préférence, l'acide acétique est utilisé.

La concentration en inhibiteur dans la composition cosmétique de l'invention peut être comprise entre 10 ppm et 30% en poids et plus préférentiellement entre 10 ppm et 15% en poids par rapport au poids total de la composition.

Pour moduler la cinétique de polymérisation anionique, on peut également augmenter la nucléophilie de la fibre par transformation chimique de la matière kératinique.

A titre d'exemple, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants:
- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

La composition de l'invention peut aussi contenir un ou plusieurs polymères ne présentant pas de réactivité sur les monomères cyanoacrylates et qui est capable d'augmenter la viscosité de la composition. L'augmentation de la viscosité permet de réduire la vitesse de polymérisation des monomères de cyanoacrylate. Pour ce faire, on peut ajouter à la composition de l'invention et de façon non exhaustive le polyméthylméthacrylate (PMMA) ou encore les copolymères à base de cyanoacrylate tels qu'ils sont décrits dans le brevet US 6,224,622.

Les compositions conformes à l'invention peuvent également contenir au moins un agent utilisé habituellement en cosmétique, choisi, par exemple, parmi des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des polymères fixants ou non, des protéines, des vitamines, des colorants directs ou d'oxydation, des agents nacrants, des propulseurs, et des épaississants minéraux ou organiques tels que le benzylidène-sorbitol et les N-acylaminoacides, les cires oxyéthylénées ou non, les paraffines, les amides gras en C10-C30 tel que le diéthanolamide laurique et leurs mélanges.

Les compositions peuvent se présenter sous différentes formes galéniques tels qu'une lotion, une mousse aérosol, un après shampooing ou un shampooing, un gel, une cire. Les compositions peuvent être contenues dans un flacon pompe, un spray aérosol. Les compositions de l'invention après application sur la chevelure peuvent être rincées ou non.

Lorsque la composition est contenue dans un aérosol, elle peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés ou non (butane, propane, isobutane) et leurs mélanges.

Selon le procédé de l'invention, la composition de l'invention est appliquée sur les fibres kératiniques, en particulier les fibres kératiniques humaines telles que les cheveux, en présence d'un agent nucléophile.

Selon un mode de réalisation particulier du procédé de l'invention, l'agent nucléophile susceptible d'initier la polymérisation du monomère électrophile peut être appliqué au préalable sur les fibres kératiniques. L'agent nucléophile peut être utilisé pur, en solution, sous forme d'une émulsion ou être encapsulé. Il peut aussi être ajouté à la composition anhydre au moment de l'emploi juste avant l'application sur les fibres kératiniques.

De préférence, cet agent nucléophile est l'eau. Cette eau peut être apportée par exemple par humidification préalable des fibres kératiniques. Elle peut aussi être ajoutée directement dans la composition avant application.

Selon un mode de réalisation particulier, il est possible de moduler la cinétique de polymérisation en humidifiant préalablement la fibre à l'aide d'une solution aqueuse dont le pH a été ajusté à l'aide d'une base, d'un acide ou d'un mélange acide/base. L'acide et/ou la base peuvent être inorganique ou organique.

Selon une variante, le procédé de traitement comprend une étape d'application d'une composition (A) comprenant des composés fluorés et éventuellement un agent nucléophile, suivie d'une étape d'application sur lesdites fibres d'au moins une composition (B), comprenant au moins un monomère électrophile, l'une et/ou l'autre des compositions contenant au moins un solvant organique liquide.

Alternativement, l'agent nucléophile peut être également dans une composition séparée.

Selon encore une autre variante, le procédé de traitement des fibres kératiniques peut être un procédé de coloration capillaire pouvant être mis en oeuvre en plusieurs étapes : une première étape qui consiste à appliquer une composition contenant le ou les pigments sur les fibres et une seconde étape qui consiste à appliquer une composition contenant entre autres un monomère électrophile, un composé fluoré et un solvant organique liquide tels que définis précédemment, l'agent nucléophile étant éventuellement présent dans la composition contenant le pigment ou dans une composition séparée.

Selon cette variante, la composition cosmétique contenant le ou les pigments est de préférence une dispersion aqueuse de pigments ce qui permet une humidification de la fibre et l'initiation de la polymérisation lorsque la composition comprenant le monomère électrophile, le composé fluoré et le solvant organique liquide est appliquée.

Selon le procédé de l'invention, un mode de réalisation préféré consiste à appliquer le monomère électrophile, les composé fluorés et éventuellement les pigments à partir d'une même composition.

Le procédé de l'invention peut comprendre des étapes additionnels intermédiaires ou finales telles que l'application d'un produit cosmétique, une étape de rinçage, une étape de séchage. Le séchage peut être effectué au casque, au sèche cheveux et/ou au fer à lisser. En particulier, l'application des compositions conformes à l'invention peut être suivie d'un rinçage.

Il est aussi possible de réaliser des applications multiples de la composition de l'invention afin d'obtenir une superposition de couches pour atteindre des propriétés spécifiques du dépôt en termes de nature chimique, résistance mécanique, épaisseur, aspect, toucher.

La présente invention concerne également l'utilisation d'une composition cosmétique telle que décrite précédemment pour le traitement des cheveux.

Lorsque la composition cosmétique ne comprend pas de pigments, la composition cosmétique peut être utilisée pour le conditionnement des cheveux.

Ainsi lorsque la composition cosmétique selon l'invention comprend à la fois le composé fluoré et un pigment, ladite composition peut être utilisée pour colorer les cheveux et éventuellement pour les conditionner.

L'invention a encore pour objet un dispositif à plusieurs compartiments ou kit, comprenant un premier compartiment, comprenant une première composition (C) comprenant le monomère électrophile et un second compartiment, comprenant une seconde composition (D) contenant au moins un composé fluoré organique à l'exception du polytétrafluoroéthylène, et des pigments enrobés de composés fluorés, l'une et/ou l'autre des compositions contenant au moins un solvant organique liquide tels que définis précédemment, l'une des deux ou les deux compositions pouvant éventuellement contenir un ou plusieurs additifs cosmétiques tels que définis précédemment.

Les exemples qui suivant sont destinés à illustrer l'invention, sans présenter un caractère limitatif.

### EXEMPLES

### Exemple 1

On a préparé une composition de coiffage selon l'invention à partir des composés suivants :

| | |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 45g |
| cyclopentadiméthylsiloxane commercialisé par Dow Corning sous le nom de DC245 Fluid | 42,75g |
| Perfluoro polyméthylisopropyléther commercialisé par Solvay sous le nom Fomblin HC 25 | 2g |
| Méthylheptylcyanoacrylate de Chemence | 10g |
| Acide acétique | 0,25g |

0,2g de la composition est appliqué sur une mèche de 1g de cheveux propre et humide. Après 15 minutes de pause, la mèche est séchée au sèche-cheveux pendant 2 minutes. On obtient une mèche dont les cheveux sont individualisés qui a un toucher agréable et rémanent.

### Exemple 2

On a préparé une composition de coiffage selon l'invention à partir des composés suivants :

| | |
|---|---|
| DC 1501 Fluid | 45g |
| DC 245 Fluid | 42,75g |
| Perfluorophenanthrene commercialisé par F2 Chemical | |
| sous le nom Flutec PC11 | 2g |
| Méthylheptylcyanoacrylate de Chemence | 10g |
| Acide acétique | 0,25 g |

0,2g de la composition est appliqué sur une mèche de 1g de cheveux propre et humide. Après 15 minutes de pause, la mèche est séchée au sèche-cheveux pendant 2 minutes. On obtient une mèche dont les cheveux sont individualisés et qui a un toucher agréable et rémanent.

### Exemple 3

On a préparé une composition de coiffage selon l'invention à partir des composés suivants :

| | |
|---|---|
| DC 1501 Fluid | 45g |
| DC 245 Fluid | 42.75g |
| Perfluorononyl diméthicone commercialisé | |
| par Phoenix Chemical sous le nom Pecosil FSL-150 | 2g |
| Méthylheptylcyanoacrylate de Chemence | 10g |
| Acide acétique | 0,25g |

0,2g de la composition est appliqué sur une mèche de 1g de cheveux propre et humide. Après 15 minutes de pause, la mèche est séchée au sèche-cheveux pendant 2 minutes. On obtient une mèche dont les cheveux sont individualisés et qui a un toucher agréable et rémanent.

### Exemple 4

On a préparé une composition de coiffage selon l'invention à partir des composés suivants :

| | |
|---|---|
| DC 245 Fluid | 84,75g |
| Perfluorononyl dimethicone commercialisé | |
| par Phoenix Chemical sous le nom Pecosil FSU-300 | 5g |
| Méthylheptylcyanoacrylate de Chemence | 10g |
| Acide acétique | 0,25 g |

0,2g de la composition est appliqué sur une mèche de 1g de cheveux propre et humide. Après 15 minutes de pause, la mèche est séchée au sèche-cheveux pendant 2 minutes. On obtient une mèche dont les cheveux sont individualisés qui a un toucher agréable et rémanent.

### Exemple 5

On a préparé une composition de coiffage selon l'invention à partir des composés suivants :

| | |
|---|---|
| DC 1501 Fluid | 45g |
| DC 245 Fluid | 42,75g |
| Trifluoropropyl dimethicone commercialisé | |
| par Dow Corning sous le nom FS-1265 fluid | 2g |
| Méthylheptylcyanoacrylate de Chemence | 10g |
| Acide acétique | 0,25g |

0,2g de la composition est appliqué sur une mèche de 1g de cheveux propre et humide. Après 15 minutes de pause, la mèche est séchée au sèche-cheveux pendant 2 minutes. On obtient une mèche dont les cheveux sont individualisés et qui a un toucher agréable et rémanent.

### Exemple 6

La composition A est réalisée à partir des ingrédients suivants :

| | |
|---|---|
| Cartafluor UH liquide commercialisé par Clariant | 10g |
| Eau | 90g |

La composition B est réalisée à partir des ingrédients suivants :

| | |
|---|---|
| DC 1501 Fluid | 45g |
| DC 245 Fluid | 44,75g |
| Méthylheptylcyanoacrylate de Chemence | 10g |
| Acide acétique | 0,25g |

0,3g de la composition A est appliquée sur une mèche de 1g de cheveux propre et humide. 0,2g de la composition B est ensuite appliqué sur cette mèche. Après 15 minutes de pause, la mèche est séchée au sèche-cheveux pendant 2 minutes.

On obtient une mèche dont les cheveux sont individualisés qui a un toucher agréable et rémanent.

### Exemple 7

On prépare la composition (A) à partir des ingrédients suivants:

| | |
|---|---|
| Foraperle 321 | |
| commercialisé par Dupont de Nemours | 20g |
| Eau | 80g |

On prépare la composition (B) à partir des ingrédients suivants:

| | |
|---|---|
| DC 1501 Fluid | 45g |
| DC 245 Fluid | 44,75g |
| Méthylheptylcyanoacrylate de Chemence | 10g |
| Acide acétique | 0,25g |

0,3g de la composition A est appliquée sur une mèche de 1g de cheveux propre et humide. 0,2g de la composition B est ensuite appliqué sur cette mèche. Après 15 minutes de pause, la mèche est séchée au sèche-cheveux pendant 2 minutes.

On obtient une mèche dont les cheveux sont individualisés et qui a un toucher agréable et rémanent.

### Exemple 8

On a préparé une composition de coloration selon l'invention à partir des composés suivants :

| | |
|---|---|
| DC 1501 Fluid | 40g |
| DC 245 Fluid | 37.75g |
| Nacre mica enrobé d'oxyde de fer brun | |
| commercialisée par Eckart sous le nom Prestige Bronze | 10g |
| Perfluoro polymethylisopropyléther | |
| commercialisé par Solvay sous le nom Fomblin HC 25 | 2g |
| Méthylheptylcyanoacrylate de Chemence | 10g |
| Acide acétique | 0,25g |

0,5g de la composition est appliqué sur une mèche de 1g de cheveux propre et humide. Après 15 minutes de pause, la mèche est séchée au sèche-cheveux pendant 2 minutes. On obtient une mèche colorée dont les cheveux sont individualisés qui a un toucher agréable et rémanent.

### Exemple 9

On a préparé une composition de coloration selon l'invention à partir des composés suivants :

| | |
|---|---|
| DC 1501 Fluid | 40g |
| DC 245 Fluid | 37,75g |
| Nacre mica enrobé d'oxyde de fer brun | |
| commercialisé par Eckart sous le nom Prestige Bronze | 10g |
| Perfluoro polymethylisopropyléther | |
| commercialisé par Solvay sous le nom Fomblin HC 25 | 2g |
| Méthylheptylcyanoacrylate de Chemence | 10g |
| Acide acétique | 0,25g |

0,5g de la composition est appliqué sur une mèche de 1g de cheveux propre et humide. Après 15 minutes de pause, la mèche est séchée au sèche-cheveux pendant 2 minutes. On obtient une mèche colorée dont les cheveux sont individualisés qui a un toucher agréable et rémanent.

### Exemple 10

On a préparé une composition de coloration selon l'invention à partir des composés suivants :

| | |
|---|---|
| DC 1501 Fluid | 40g |
| DC 245 Fluid | 37,75g |
| Nacre mica enrobé d'oxyde de fer brun | |
| commercialisé par Eckart sous le nom Prestige Bronze | 10g |
| Perfluorophenanthrene | |
| commercialisé par F2 Chemical sous le nom Flutec PC11 | 2g |
| Méthylheptylcyanoacrylate de Chemence | 10g |
| Acide acétique | 0,25g |

0,5g de la composition est appliqué sur une mèche de 1g de cheveux propre et humide. Après 15 minutes de pause, la mèche est séchée au sèche-cheveux pendant 2 minutes. On obtient une mèche colorée dont les cheveux sont individualisés qui a un toucher agréable et rémanent.

### Exemple 11

On a préparé une composition de coloration selon l'invention à partir des composés suivants :

| | |
|---|---|
| DC 1501 Fluid | 40g |
| DC 245 Fluid | 37,75g |
| Nacre mica enrobé d'oxyde de fer brun | |
| commercialisée par Eckart sous le nom Prestige Bronze | 10g |
| Perfluorononyl dimethicone commercialisé | |
| par Phoenix Chemical sous le nom Pecosil FSL-150 | 2g |
| Méthylheptylcyanoacrylate de Chemence | 10g |
| Acide acétique | 0,25g |

0,5g de la composition est appliqué sur une mèche de 1g de cheveux propre et humide. Après 15 minutes de pause, la mèche est séchée au sèche-cheveux pendant 2 minutes. On obtient une mèche colorée dont les cheveux sont individualisés qui a un toucher agréable et rémanent.

### Exemple 12

On a préparé une composition de coloration selon l'invention à partir des composés suivants :

| | |
|---|---|
| DC 1501 Fluid | 40g |
| DC 245 Fluid | 37,75g |
| Nacre mica enrobé d'oxyde de fer brun | |
| commercialisée par Eckart sous le nom Prestige Bronze | 10g |
| Trifluoropropyl dimethicone commercialisé | |
| par Dow Corning sous le nom FS-1265 fluid | 2g |
| Méthylheptylcyanoacrylate de Chemence | 10g |
| Acide acétique | 0,25g |

0,5g de la composition est appliqué sur une mèche de 1g de cheveux propre et humide. Après 15 minutes de pause, la mèche est séchée au sèche-cheveux pendant 2 minutes. On obtient une mèche colorée dont les cheveux sont individualisés qui a un toucher agréable et rémanent.

### Exemple 13

On prépare la composition (A) à partir des ingrédients suivants:

| | |
|---|---|
| Cartafluor UH liquide commercialisé par Clariant | 10g |
| Eau | 90g |

On prépare la composition (B) à partir des ingrédients suivants:

| | |
|---|---|
| DC 1501 Fluid | 40g |
| DC 245 Fluid | 39.75g |
| Nacre mica enrobé d'oxyde de fer brun | |
| commercialisé par Eckart sous le nom Prestige Bronze | 10g |
| Méthylheptylcyanoacrylate de Chemence | 10g |
| Acide acétique | 0,25g |

0,3g de la composition A est appliquée sur une mèche de 1g de cheveux propre et humide. 0,5g de la composition B est ensuite appliqué sur cette mèche. Après 15 minutes de pause, la mèche est séchée au sèche-cheveux pendant 2 minutes. On obtient une mèche colorée dont les cheveux sont individualisés qui a un toucher agréable et rémanent.

## Revendications

1. Composition cosmétique de traitement pour les fibres kératiniques, en particulier des fibres kératiniques humaines telles que les cheveux, **caractérisée par le fait qu'**elle comprend au moins un solvant organique liquide, au moins un monomère électrophile, et au moins un composé fluoré organique contenant au moins 4 atomes de carbone, à l'exception du polytétrafluoroéthylène et des pigments enrobés de composés fluorés.

2. Composition cosmétique selon la revendication 1 dans laquelle le ou les monomères électrophile sont des monomères de formule (I) : 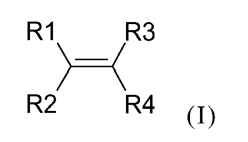 dans laquelle :
- R₁ et R₂ désignent chacun, indépendamment l'un de l'autre, un groupe peu ou non électro-attracteur, et
- R₃ et R₄ désignent chacun, indépendamment l'un de l'autre, un groupe électro-attracteur.

3. Composition cosmétique selon la revendication 2, caractérsiée par le fait que les monomères de formule (I) sont tels que R₁ et R₂, désignent chacun, indépendamment l'un de l'autre, un atome d'hydrogène, un groupe hydrocarboné saturé ou non, linéaire, ramifié ou cyclique, comportant de préférence de 1 à 20 atomes de carbones, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène, R désignant un groupe hydrocarboné saturé ou non, linéaire, ramifié ou cyclique, comportant de 1 à 20 atomes de carbones, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère, R' étant un radical alkyle en C₁-C₁₀.

4. Composition cosmétique selon la revendication 2 ou 3, **caractérisée par le fait que** les monomères de formule (A) sont tels que R₃ et R₄, indépendamment l'un de l'autre sont choisis parmi les groupements parmi les groupements -N(R)₃⁺, -S(R)₂⁺, -SH₂⁺, -NH₃⁺, - NO₂, -SO₂R, -C≡N, -COOH, -COOR, -COSR, -CONH₂, CONHR, -F, - Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle ou aryloxy, R désignant un groupe hydrocarboné saturé ou non, linéaire, ramifié ou cyclique, comportant de 1 à 20 atomes de carbones, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère, R' étant un radical alkyle en C₁-C₁₀.

5. Composition cosmétique selon la revendication 1 ou 2, **caractérisée par le fait que** les monomères sont des monomères cyanoacrylates de formule (II): 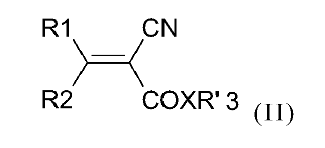 X désignant NH, S ou O,
R'₃ étant choisi parmi un atome d'hydrogène ou R,
R, R₁ et R₂ étant tel que défini à la revendication 3.

6. Composition cosmétique selon l'une quelconque des revendications 2 à 5, **caractérisée par le fait que** le monomère électrophile est tel que R₁ et R₂ représentent un atome d'hydrogène.

7. Composition cosmétique selon l'une quelconque des revendications 1 à 6, **caractérisée par le fait que** les monomères cyanoacrylates correspondent à la formule (V) : 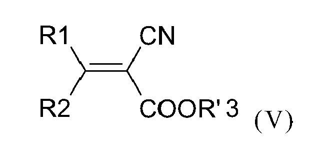 dans laquelle R'3 représente un radical alkyle en C₁-C₁₀, alcoxy(C₁-C₄) alkyle(C₁-C₁₀) ou alcényle en C2-C10 et R₁ et R₂ sont tels que définis précédemment.

8. Composition cosmétique selon la revendication 7 **caractérisée par le fait que** R'3 est un radical alkyle comprenant de 6 à 10 atomes de carbone.

9. Composition cosmétique selon la revendication 7, **caractérisée par le fait que** R1 et R2 représentent un atome d'hydrogène.

10. Composition cosmétique selon l'une quelconque des revendications précédentes, **caractérisée par le fait que** le monomère électrophile est un cyanoacrylate d'alkyle de formule 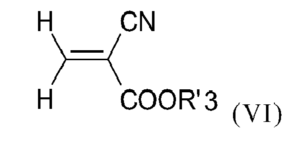
dans laquelle : R'₃ = -(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

11. Composition cosmétique selon l'une quelconque des revendications précédentes, **caractérisée par le fait que** le ou les monomères cyanoacrylates sont fixés de façon covalente sur des supports tels que des polymères, des oligomères ou des dendrimères.

12. Composition cosmétique selon l'une quelconque des revendications précédentes, **caractérisée par le fait que** le composé fluoré est choisi parmi les composés fluorosiliconés de formule générale (VII) : 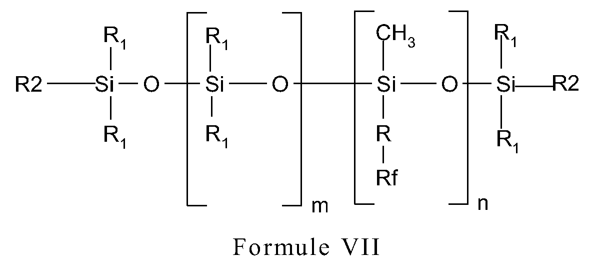 dans laquelle :
- R représente un groupement divalent alkyle linéaire ou ramifié, ayant 1 à 6 atomes de carbone, de préférence un groupement divalent méthyle, éthyle, propyle ou butyle,
- Rf représente un radical fluoroalkyle, notamment un radical perfluoroalkyle, ayant 1 à 12 atomes de carbone, de préférence 1 à 9 atomes de carbone,
- R₁ représente, indépendamment l'un de l'autre, un radical alkyle en C₁-C₂₀, un radical hydroxyle, un radical phényle,
- R₂ représente Rₗ ou R_{f}
- m est choisi de 0 à 500, de préférence de 0 à 200, et
- n est choisi de 1 à 1000, de préférence de 1 à 500.

13. Composition cosmétique selon la revendication 11, **caractérisée par le fait que** les groupements R₁ sont identiques et représentent un radical méthyle

14. Composition cosmétique selon l'une quelconque des revendications 1 à 11, **caractérisée par le fait que** le composé fluoré est choisi parmi les perfluorocycloalkyles correspondant à la formule générale (VIII) suivante : 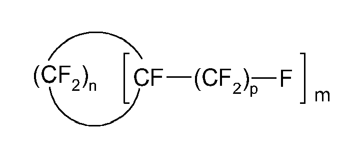 dans laquelle n est égal à 3, 4 ou 5, m est égal à 1 ou 2, et p est égal à 0, 1, 2 ou 3 ; sous réserve que lorsque m=2, les groupements n'étant pas nécessairement en alpha l'un par rapport à l'autre.

15. Composition cosmétique selon l'une quelconque des revendications 1 à 11, **caractérisée par le fait que** le composé fluoré est choisi parmi les composés fluoroalkyles ou hétérofluoroalkyles répondant à la formule (IX) suivante :
CH₃-(CH₂)ₙ-[Z]ₜ-X-CF₃ (IX)
dans laquelle t est 0 ou 1 ; n est 0, 1, 2 ou 3 ; X est un radical perfluoroalkyle divalent, linéaire ou ramifié, ayant de 2 à 5 atomes de carbone, et Z représente O, S, ou NR, R étant hydrogène, un radical - (CH₂)ₙ-CH₃ ou -(CF₂)ₘ-CF₃, m étant 2, 3, 4 ou 5.

16. Composition cosmétique selon l'une quelconque des revendications 1 à 11, **caractérisée par le fait que** le composé fluoré est choisi parmi les perfluoroalcanes répondant à la formule (X) suivante :
CF₃-(CF₂)ₙ-CF₃
dans laquelle n vaut de 2 à 16, de préférence de 2 à 6.

17. Composition cosmétique selon l'une quelconque des revendications 1 à 11, **caractérisée par le fait que** le composé fluoré est choisi parmi les perfluoropolyéthers répondant aux formules (XI) et (XII) suivantes : 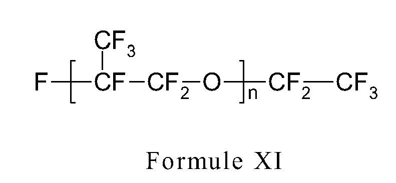 dans laquelle n vaut de 7 à 30 ; et 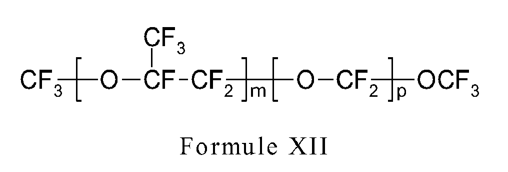 le rapport m/p étant de 20 à 40, et le poids moléculaire allant de 500 à 20000.

18. Composition cosmétique selon l'une quelconque des revendications 1 à 11, **caractérisée par le fait que** le composé fluoré est choisi parmi les dérivés de perfluomorpholine répondant à la formule (XIII) suivante : 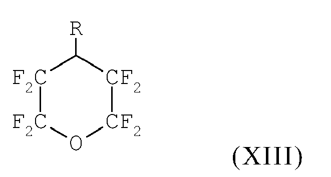 dans laquelle R représente un radical perfluoroalkyle en C₁-C₄.

19. Composition cosmétique selon l'une quelconque des revendications 1 à 11, **caractérisée par le fait que** le composé fluoré est choisi parmi les esters fluorés répondant à la formule (XIV) suivante : 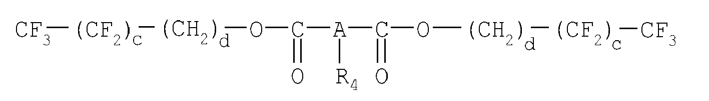 dans laquelle :
R₄ représente un atome d'hydrogène ou le radical 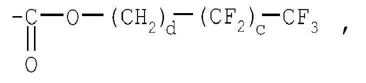 A représente une chaîne alkylène ou alcénylène, linéaire ou ramifiée, éventuellement hydroxylée, en C₁-C₁₈,
c est 1 à 17, et
d est 1 à 18.

20. Composition cosmétique selon l'une quelconque des revendications 1 à 11, **caractérisée par le fait que** le composé fluoré est choisi parmi les copolymères à base de perfluoroalkylpolyacrylate, susceptible d'être obtenu par polymérisation à partir :
(i) d'un monomère de type acrylique ou de type acrylamide présentant la formule (A') suivante : 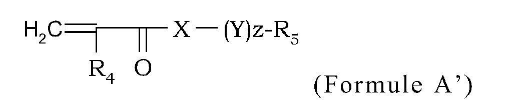 dans laquelle :
- R₄ représente un atome d'hydrogène ou un radical alkyle en C₁-C₄,
- X représente O ou -N-R₂, pour lequel R₂ représente un atome d'hydrogène ou un radical alkyle ou hydroxyalkyle en C₁-C₁₀.
- Y représente un radical alkylène en C₁-C₆
- z est égal à 0 ou 1,
- R₅ représente un radical alkyle linéaire ou ramifiée en C₁-C₂₀ dont tout ou partie des atomes d'hydrogène est remplacé par des atomes de fluor ou un radical contenant des groupements perfluoropolyéthers ;
(ii) d'un monomère de type B' répondant à la formule (B') : 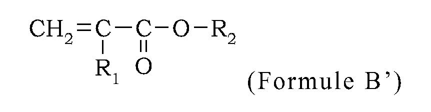 dans laquelle :
- R₁ représente un atome d'hydrogène ou un radical alkyle en C₁-C₄ et
- R₂ représente un radical alkyle en C₁-C₂₀, de préférence C₂-C₈, un radical hydroxycarboné en C₂-C₆, de préférence C₂-C₄, ou un radical -(CH₂)ₙ-NH-R₃ avec R₃ représentant un alkyle en C₁-C₆ ou un cycloalkyle et n étant un entier allant de 1 à 4.
(iii) d'un monomère de type C comprenant un motif alkyl(méth)acrylamide répondant à la formule C' : 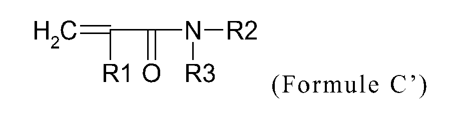 dans laquelle R¹ représente H ou CH₃ ;
R² et R³, identiques ou différents, représentent H, un radical hydrocarboné linéaire ou ramifié, saturé ou insaturé, en C₁-C₄₀, éventuellement substitué par un ou plusieurs groupes hydroxyle, acide carboxylique, acide sulfonique ou acide phosphonique, estérifié ou non, un groupe cycloalkyle en C₅-C₁₀ ou aryle en C₅-C₁₀, éventuellement substitué par un ou plusieurs groupes alkyle, linéaires ou ramifiés, en C₁-C₄, hydroxyle, acide carboxylique ou acide sulfonique ou pouvant comporter, à ses extrémités ou à l'intérieur, un ou plusieurs hétéroatomes choisis parmi O, S, N, Si et P ou un groupe carbonyle, ou pouvant comporter un ou plusieurs hétéroatomes choisis parmi O, S et N ;

21. Composition cosmétique selon l'une quelconque des revendications 1 à 11, **caractérisée par le fait que** le composé fluoré est choisi parmi les composés fluorés contenant la sous-unité suivante : 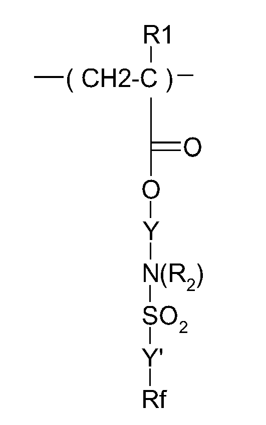 dans laquelle :
- R₁ représente un atome d'hydrogène ou un radical alkyle en C₁-C₄,
- R₂ représente un atome d'hydrogène ou un radical alkyle ou hydroxyalkyle en C₁-C₁₀.
- Y et Y' représentent des radicaux alkylène en C₁-C₆
- Rf représente un radical alkyle linéaire ou ramifiée en C₁-C₂₀ dont tout ou partie des atomes d'hydrogène est remplacé par des atomes de fluor ou un radical contenant des groupements perfluoropolyéthers.

22. Composition cosmétique selon l'une quelconque des revendications 1 à 11, **caractérisée par** le fait le composé fluoré est choisi parmi les fluorohydrocarbures.

23. Composition cosmétique selon l'une quelconque des revendications précédentes, **caractérisée par le fait que** le composé fluoré est choisi parmi les composés fluorosiliconés, les perfluorocycloalkyles, les perfluoropolyéthers, les copolymères à base de perfluoroalkylpolyacrylate.

24. Composition cosmétique selon l'une quelconque des revendications précédentes, **caractérisée par le fait que** le solvant organique liquide est choisi parmi les alcools aromatiques tels que l'alcool benzylique ; les alcools gras liquides , notamment en C₁₀-C₃₀; les polyols ; les silicones volatiles, les polydiméthylsiloxanes modifiées ou non par des fonctions alkyle et/ou amine et/ou imine et/ou fluoroalkyl et/ou carboxylique et/ou betaïne et/ou ammonium quaternaire, les polydiméthylsiloxanes modifiées liquides, les huiles minérales, organiques ou végétales, les alcanes ; les acides gras liquides, les esters gras liquides et leurs mélanges.

25. Composition cosmétique selon la revendication 24, **caractérisée par le fait que** le solvant organique liquide est constitué d'un mélange de poly diméthyl siloxane alpha-omega dihydroxylé/cyclopentadiméthylsiloxane et de cyclopentadiméthyl siloxane.

26. Composition cosmétique selon la revendication 24 ou 25, **caractérisée par le fait que** le solvant organique représente plus de 50 à 99 % en poids, par rapport au poids total de la composition cosmétique.

27. Composition cosmétique selon l'une quelconque des revendications précédentes, **caractérisée par le fait qu'**elle comprend en outre un ou des pigments minéraux ou organiques, de préférence des nacres et des pigments composites.

28. Composition cosmétique selon l'une quelconque des revendications précédentes, **caractérisée par le fait que** ladite composition est anhydre.

29. Composition cosmétique selon l'une quelconque des revendications précédentes, **caractérisée par le fait qu'**elle comprend en outre au moins un agent nucléophile.

30. Composition cosmétique selon les revendications 1 à 27 et 29, **caractérisée par le fait que** l'agent nucléophile est de l'eau.

31. Procédé de traitement cosmétique des fibres kératiniques, **caractérisé par le fait que** l'on applique sur lesdites fibres la composition telle que définie à l'une quelconque des revendications 1 à 28 en présence d'un agent nucléophile.

32. Procédé de traitement selon la revendication 31, **caractérisé par le fait que** l'on applique la composition telle que définie à la revendication 29 ou 30.

33. Procédé de traitement selon la revendication 31, **caractérisée par le fait que** l'on applique une composition (A) comprenant des composés fluorés et éventuellement un agent nucléophile, suivie d'une étape d'application sur lesdites fibres d'au moins une composition (B), comprenant au moins un monomère électrophile, l'une et/ou l'autre des deux compositions contenant au moins un solvant organique liquide.

34. Procédé de coloration capillaire selon la revendication 30, **caractérisé par le fait qu'**il comprend une première étape consistant à appliquer une composition contenant au moins un pigment tel que défini à la revendication 27 sur lesdites fibres kératiniques et une seconde étape consistant à appliquer une composition cosmétique telle que définie à l'une quelconque des revendications 1 à 26 et 28, l'agent nucléophile étant présent dans la composition comprenant le pigment ou dans une composition séparée.

35. Utilisation d'une composition cosmétique selon l'une quelconque des revendications 1 à 30 pour le traitement des cheveux.

36. Dispositif à plusieurs compartiments ou kit, comprenant un premier compartiment, comprenant une première composition (C) comprenant ledit monomère électrophile et un second compartiment, comprenant une seconde composition (D) contenant au moins un composé fluoré organique contenant au moins 4 atomes de carbone à l'exception du polytétrafluoroéthylène et des pigments enrobés de composés fluorés, l'une et/ou l'autre des compositions contenant au moins un solvant organique liquide et éventuellement, l'un des deux ou les deux compositions pouvant contenir un ou plusieurs additifs cosmétiques tels que définis précédemment.
